# EUROPÄISCHE PATENTSCHRIFT

(11) **EP 1 054 879 B1**
(45) Veröffentlichungstag und Bekanntmachung des Hinweises auf die Patenterteilung: **21.11.2001**
(21) Anmeldenummer: 99907435.4
(22) Anmeldetag: 27.01.1999
(51) Int. Cl.: C07D 301/08, B01J 23/52, B01J 23/66, C07D 301/10

(54) **VERFAHREN ZUR KATALYTISCHEN DIREKTOXIDATION UNGESÄTTIGTER KOHLENWASSERSTOFFE IN DER GASPHASE**
METHOD FOR THE DIRECT CATALYTIC OXIDATION OF UNSATURATED HYDROCARBONS IN GASEOUS PHASE
PROCEDE D'OXYDATION DIRECTE PAR VOIE CATALYTIQUE D'HYDROCARBURES INSATURES DANS LA PHASE GAZEUSE

(30) Priorität: 06.02.1998 DE 19804709
(43) Veröffentlichungstag der Anmeldung: 29.11.2000
(73) Patentinhaber: Bayer Aktiengesellschaft, 51368 Leverkusen (DE)
(72) Erfinder: WEISBECK, Markus, D-51465 Bergisch Gladbach (DE); DORF, Ernst-Ulrich, D-47802 Krefeld (DE); WEGENER, Gerhard, D-40822 Mettmann (DE); SCHILD, Christoph, D-51373 Leverkusen (DE); LÜCKE, Bernhard, D-10179 Berlin (DE); DILCHER, Herbert, D-15834 Rangsdorf (DE); SCHÜLKE, Ulrich, D-12623 Berlin (DE)
(86) Internationale Anmeldenummer: EP9900530
(87) Internationale Veröffentlichungsnummer: WO9940077

(56) Entgegenhaltungen:
- EP-A- 0 709 360
- EP-A- 0 850 936
- WO-A-97/47386
- WO-A-98/00413
- FR-A- 2 771 310
- S. TSUBOTA ET AL.: ACS SYMP. SER., Bd. 552, 1994, Seiten 420-428, XP002114494 -& CHEMICAL ABSTRACTS, vol. 120, no. 16, 18. April 1994 (1994-04-18) Columbus, Ohio, US; abstract no. 201527, XP002114498
- M. HARUTA ET AL.: RES. CHEM. INTERMED., Bd. 24, Nr. 3, 1998, Seiten 329-336, XP002114495
- T. HAYASHI ET AL.: J. CATAL., Bd. 178, Nr. 2, 1998, Seiten 566-575, XP002114496
- B.S. UPHADE ET AL.: CHEM. LETT., Bd. 12, 1998, Seiten 1277-1278, XP002114497

## Beschreibung

Die Erfindung betrifft ein katalytisches Gasphasenverfahren zur Herstellung von Epoxiden aus ungesättigten Kohlenwasserstoffen durch Oxidation mit molekularem Sauerstoff in Gegenwart von molekularem Wasserstoff sowie mit nanoskaligen Goldpartikeln belegte Katalysatoren für dieses Verfahren.

Im allgemeinen verlaufen Direktoxidationen von ungesättigten Kohlenwasserstoffen mit molekularem Sauerstoff in der Gasphase - selbst in Gegenwart von Katalysatoren - nicht unterhalb von 200°C, und es ist daher schwierig, oxidationsempfindliche Oxidationsprodukte, wie z. B. Epoxide, Alkohole oder Aldehyde, selektiv herzustellen, da die Folgereaktionen dieser Produkte häufig schneller als die Oxidation der eingesetzten Olefine selbst verlaufen.

Propenoxid stellt eine der wichtigsten Grundchemikalien der Chemischen Industrie dar. Das Einsatzgebiet liegt mit einem Anteil von über 60 % im Kunststoffsektor, speziell zur Herstellung von Polyetherpolyolen für die Synthese von Polyurethanen. Daneben werden noch größere Marktanteile im Bereich der Glykole, besonders bei den Schmier- und Frostschutzmitteln, von den Propenoxid-Derivaten belegt.

Heute werden weltweit etwa 50% des Propenoxids über das "Chlorhydrin-Verfahren" synthetisiert. Weitere 50%, mit steigender Tendenz, liefern die "Oxiran-Verfahren.

Beim Chlorhydrinverfahren (F. Andreas et al.; Propylenchemie, Berlin 1969) wird durch Reaktion von Propen mit HOC1 (Wasser und Chlor) zuerst das Chlorhydrin und anschließend hieraus durch Abspaltung von HCI mit einer Base das Propenoxid gebildet. Das Verfahren ist kostenintensiv, weist aber bei entsprechender Optimierung eine hohe Selektivität (> 90 %) bei hohen Umsätzen auf. Der Chlorverlust beim Chlorhydrin-Verfahren in Form wertloser Calciumchlorid- bzw. Natriumchlorid-Lösungen und die damit verbundene hohe Salzfracht im Abwasser hat frühzeitig zur Suche nach chlorfreien Oxidationssystemen geführt.

Die Oxidationsverfahren verwenden anstelle des anorganischen Oxidationsmittels HOCI organische Verbindungen zur Übertragung von Sauerstoff auf Propen. Diese indirekte Epoxidation beruht auf der Tatsache, daß organische Peroxide wie Hydroperoxide in flüssiger Phase ihren Peroxidsauerstoff selektiv auf Olefine unter Bildung von Epoxiden übertragen können. Die Hydroperoxide gehen dabei in Alkohole, die Peroxycarbonsäuren in Säuren über. Hydroperoxide werden durch Autoxidation mit Luft oder molekularen Sauerstoff aus dem entsprechenden Kohlenwasserstoff erzeugt. Ein gravierender Nachteil der indirekten Oxidation ist die wirtschaftliche Abhängigkeit des Propenoxidwertes vom Marktwert des Koppelproduktes sowie die kostenintensive Herstellung der Oxidationsmittel.

Mit Titansilicalit (TS 1) als Katalysator (Notari et al., US 44 10 501 und US 47 01 428) war es erstmalig möglich, Propen mit Wasserstoffperoxid in der Flüssigphase unter sehr milden Reaktionsbedingungen mit Selektivitäten > 90 % zu epoxidieren (Clerici et al., EP-A 230 949).

Die Propenoxidation gelingt mit geringer Ausbeute in der Flüssigphase an platinmetallhaltigen Titansilikaliten auch mit einem Gasgemisch bestehend aus molekularem Sauerstoff und molekularem Wasserstoff (JP-A 92/352771).

In der US 5 623 090 (Haruta et al.) wird erstmalig eine Gasphasen-Direktoxidation von Propen zu Propenoxid mit 100%iger Selektivität beschrieben. Es handelt sich hierbei um eine katalytische Gasphasenoxidation mit molekularem Sauerstoff in Gegenwart des Reduktionsmittels Wasserstoff. Als Katalysator wird handelsübliches Titandioxid verwendet, das mit nanoskaligen Goldteilchen belegt ist. Unter nanoskalige Goldteilchen werden dabei Teilchen mit einem Durchmesser im nm-Bereich verstanden. Der Propenumsatz und die Propenoxidausbeute werden mit maximal 2,3 % angegeben. Die beschriebenen Au/TiO₂-Katalysatoren erreichen den ca. 2%igen Propenumsatz nur für sehr kurze Zeit; z. B. sind die typischen Halbwertszeiten bei moderaten Temperaturen (40-50°C) noch unbefriedigend (Haruta et al., 3 rd World Congress on Oxidation Catalysis 1997, S. 965-970, Fig. 6). Dieses Verfahren hat also den Nachteil, daß die ohnehin geringe Epoxidausbeute durch schnelle Desaktivierung weiter stark herabgesetzt wird.

Für eine wirtschaftliche Nutzung ist daher die Entwicklung von Katalysatoren mit deutlich besseren Anfangsaktivitäten bei stark erhöhter Katalysatorstandzeit unbedingt weiter notwendig.

Gegenstand der Erfindung ist daher ein Verfahren zur Oxidation ungesättigter Kohlenwasserstoffe in der Gasphase in Gegenwart eines Wasserstoff-Sauerstoffgemisches gegebenenfalls unter Zusatz eines Inertgases an einem mit Goldteilchen belegten Trägerkatalysator, dadurch gekennzeichnet, daß ein mit nanoskaligen Goldteilchen belegter gegebenenfalls dotierten enthaltenden Titanoxidhydrat hergestellter und kalzinierter Katalysator eingesetzt wird.

Das erfindungsgemäße Verfahren kann auf alle Olefine angewendet werden. Da die Gasphasenoxidation aufgrund höherer erreichbarer Selektivitäten zweckmäßig bei niedrigen Temperaturen (<120°C) stattfindet, können alle ungesättigten Kohlenwasserstoffe oxidiert werden, aus denen solche Oxidationsprodukte gebildet werden, deren Partialdruck niedrig genug liegen, um das Produkt ständig vom Katalysator zu entfernen. Bevorzugt sind ungesättigte Kohlenwasserstoffe mit bis zu zwölf Kohlenstoffatomen, insbesondere Ethen, Propen, 1-Buten oder 2-Buten.

Die Katalysatorpräparation hat entscheidenden Einfluß auf die Katalysatoraktivität. Bevorzugt werden die Katalysatoren dabei nach der "deposition-precipitation"-Methode hergestellt. Hierbei wird zu einer gerührten wäßrigen Suspension des als Katalysatorträger verwendeten Titanoxidhydrats eine wäßrige Lösung einer anorganischen oder organischen Goldverbindung tropfenweise zugesetzt. Bevorzugt wird ein wasserenthaltendes Lösungsmittel verwendet. Andere Lösungsmittel wie z. B. Alkohole können ebenfalls eingesetzt werden. Versetzt man diese Gold(III)-Salzlösung mit Basen (z. B. Natriumcarbonat oder Alkali- bzw. Erdalkalilauge) bis zu einem pH-Wert von 7-8,5, so fällt Gold in Form von Au(III) Chlorohydroxo- bzw. Oxohydroxokomplexen oder als Goldhydroxid auf der Titanoxidhydratoberfläche aus. Um eine gleichförmige Ablagerung von ultrafeinen Goldteilchen herbeizuführen, muß die Veränderung des pH-Wertes durch eine langsame tropfenweise Zugabe dieser alkalischen wäßrigen Lösung gesteuert werden. Da im Überschuß von Alkalilauge sich die abgeschiedenen Goldverbindungen unter Bildung von Auraten ([Au(OH)₄]⁻ bzw AuO₂⁻) wieder auflösen, muß aus diesem Grund ein pH-Wert zwischen 7-8,5 eingestellt werden.

Ausgefälltes Gold(III)hydroxid ist als solches nicht isolierbar, sondern geht beim Trocknen in das Metahydroxid AuO(OH) bzw. Au₂O₃ über, welches sich bei der Kalzinierung oberhalb von 150 °C unter Sauerstoffabgabe zu elementarem Gold zersetzt. Die so generierten nanoskaligen Goldteilchen sind auf der Trägeroberfläche festhaftend immobilisiert und weisen Teilchendurchmesser < 10, bevorzugt < 6 nm auf. Die Goldmenge, die auf den Träger aufgebracht wird, hängt von verschiedenen Variablen ab, so z.B. von der Oberfäche, von der Porenstruktur und von der chemischen Oberflächenbeschaffenheit des Trägers. Die Eigenschaften des Trägers spielen somit für die katalytische Wirkung eine wichtige Rolle.

Überraschend wurde gefunden, daß beim Einsatz amorpher oberflächenreicher, hydratisierter Titanoxidhydrate zur Belegung mit Gold die katalytischen Aktivitäten bei der Epoxidierung von Propen zu Propenoxid drastisch höher liegen. Diese eingesetzten Titanoxidhydrate haben Wassergehalte von 5 bis 50 Gew.-%, und Oberflächen > 50 m²/g. Mit einem auf der Basis von Titanoxidhydrat präparierten Katalysator, der 0,5 Gew.-% Gold enthält, werden z.B. Propenoxid-Anfangsausbeuten > 4 % erhalten.

Der Wassergehalt der eingesetzten Titanoxidhydrate liegt üblicherweise zwischen 5 und 50 Gew.-%, bevorzugt zwischen 7-20 Gew.-%. Gold wird bei der Katalysatorpräparation auf Titanoxidhydrat in einem Fällungsschritt in Form von Au(III)-Verbindungen aufgebracht. Der auf diese Weise beladene Träger zeigt aber noch keine katalytische Aktivität. Erst eine Kalzinierung im Luftstrom bei 350 bis 500°C macht aus dieser Vorstufe ein katalytisch aktives Material.

Geringe Sulfatgehalte in den TiO(OH)ₙ-Vorstufen bewirken überraschenderweise eine drastische Verbesserung der katalytischen Eigenschaften der damit hergestellten Katalysatoren. Bevorzugt werden daher Katalysatoren auf der Basis von Titanoxidhydrat mit einem Sulfatgehalt zwischen 0,1 und 6 Gew.-%, bevorzugt 0,2-1 Gew.-% eingesetzt. Das Sulfat kann bei der Titanoxidhydrat-Herstellung zugesetzt werden oder nachträglich durch Behandlung der Titanoxidhydrate mit Reagenzien (z.B. Schwefelsäure oder Natriumsulfat).

Die Konzentration der löslichen Goldverbindung in der gerührten Suspension hat einen deutlichen Einfluß auf die katalytische Aktivität des daraus hergestellten Katalysators. Durch eine mehrfache Wiederholung des Fällungsvorgangs mit geringen Goldmengen (z.B. jeweils mit 0,5 Gew.-% Gold) lassen sich Katalysatoren mit deutlich erhöhten katalytischen Aktivitäten im Vergleich zu Katalysatoren mit gleich hoher Gold-Beladung, die in einem Schritt aufgebracht wurde, herstellen. Bevorzugt werden daher im erfindungsgemäßen Verfahren Katalysatoren eingesetzt, bei denen nach dem beschriebenen "deposition-precipitation"-Verfahren nach Waschung und Trocknung wiederholt auf den Träger Goldmengen zwischen 0,05 bis 10 Gew.-%, bevorzugt 0,1 bis 1 Gew.-%, aufgebracht wurden. Der erfindungsgemäß so hergestellte Katalysator ergibt beim Einsatz in der Direktoxidation von Propen mit molekularem Sauerstoff in Gegenwart von molekularem Wasserstoff Propenoxid mit Ausbeuten von >4 % bei Selektivitäten >97 %.

Bei der Katalysatorpräparation erfolgt die Reduktion der auf der Oberfläche gefällten Goldhydroxide während der Kalzinierung. Setzt man ausgewählte Reduktionsmittel (z. B. Natriumcitrat, Magnesiumcitrat,..) während der Katalysatorpräparation zu, so lassen sich die katalytischen Aktivitäten geringfügig steigern.

Die spezifische synergistische Wechselwirkung zwischen dem nanoskaligen Gold und TiO(OH)ₙ-Träger werden auch dann erreicht, wenn die beiden Komponenten auf zusätzliche andere Träger (z. B. SiO₂, Al₂O₃, ZnO.) aufgebracht werden.

Die Mengen an eingesetztem Katalysator und der eingesetzten Gasmengen sind nicht begrenzt. Üblicherweise sollte die "space velocity" des Gasstromes durch das Katalysatorbett ca. 0,5 bis 20 l/g Kat. x h⁻¹ betragen.

Das erfindungsgemäße Verfahren wird in Gegenwart der Gase Sauerstoff und Wasserstoff gegebenenfalls unter Zusatz von Inertgas durchgeführt. In Gegenwart dieser Gase werden bei 150°C neben den Hauptprodukten Wasser, Propan und CO₂ auch die Oxygenate Propenoxid und Aceton gefunden. Bei einer Temperatur zwischen 30-60°C werden neben dem Hauptprodukt Propylenoxid (ca. 4 % Ausbeute) nur noch Wasser und Spuren anderer Komponenten (ca. 1 % bezogen auf PO) gefunden.

Die Zusammensetzung der Gasphase, bestehend aus Propen, Sauerstoff, Wasserstoff und eventuell einem Inertgas ist nicht nur für die Raum-Zeit-Ausbeute wichtig, sondern auch für die Sicherheit. Theoretisch können alle molaren Zusammensetzungen der Gase Propen/ Sauerstoff/Wasserstoff/Stickstoff eingesetzt werden. Gasmischungen aus Sauerstoff und Wasserstoff sind bekannterweise in bestimmten Zusammensetzungen explosiv (Knallgas). Überraschenderweise wurde gefunden, daß die oben beschriebene Oxidationsreaktion annähernd unter "Hydrierbedingungen" außerhalb der Explosionsgrenzen durchgeführt werden kann. "Hydrierbedingungen" bedeutet, daß neben einem Überschuß an Wasserstoff nur sehr geringe Mengen an Sauerstoff eingesetzt werden. Daher werden für die Oxidationsreaktion folgende Gaserhältnisse: H₂ / Kohlenwasserstoff / Sauerstoff Stickstoff: 20-80 Vol-% / 10 - 50 Vol-% / 1 -10 Vol-% / 0-50 Vol-% eingesetzt. Bevorzugt H₂ / Kohlenwasserstoff / Sauerstoff / Stickstoff: 30-75 % / 15- 40 / 3-8 % / 0-10 %. Der molekulare Sauerstoff, der für die Reaktion eingesetzt wird, kann vielfältiger Herkunft sein, z. B. reiner Sauerstoff, Luft oder andere Sauerstoff/Inertgasmischungen.

Neben dem erfindungsgemäßen Verfahren zur Oxidation ungesättigter Kohlenwasserstoffe ist ein weiterer Gegenstand der Erfindung auch der in diesem Verfahren eingesetzte Katalysator.

### Beispiele

### Direktoxidation von Propen zu Propenoxid

**Standardreaktionsbedingungen:** Bei dem Reaktor handelt es sich um einen Festbettrohrreaktor (1 cm Durchmesser, 20 cm Länge) aus doppelwandigem Glas, der mittels eines Wasser-Thermostaten auf 46°C temperiert wird. Dem Reaktor ist eine statische Misch- und Temperierstrecke vorgeschaltet. Der Gold-Trägerkatalysator wird auf einer Glasfritte vorgelegt. Die Katalysatorbelastung beträgt 1,8 l / g Kat. h. Die Eduktgase werden mittels Massendurchflußregler in den Reaktor von oben nach unten eindosiert. Die Eduktgasverhältnisse sind O₂ / H₂ / C₃H₆ : 0,1 / 1,3 / 0,4 l/h. Das Reaktionsgasgemisch wird mittels Gaschromatographie mit einem FID- (alle sauerstoffhaltigen organischen Verbindungen, Ausnahme CO₂) und WLD-Detektor (Permanentgase, CO, CO₂, H₂O) analysiert. Die Anlage wird über ein zentrales Meßwerterfassungssystem gesteuert.

Für die Reaktionen unter Druck wird ein analoger Festbettrohrreaktor aus V4A mit nachgeschaltetem Druckhalteventil eingesetzt.

Alle Katalysatoren werden mit TEM (Transmission Elctron Microscopy) hinsichtlich der Goldpartikelgröße untersucht.

In Beispiel 20 wird Ethylen anstelle von Propen eingesetzt.

In Beispiel 21 wird 1-Buten anstelle von Propen eingesetzt.

### Beispiel 1

Zur Suspension von 10 g Titanoxidhydrat (BET-Oberfläche von 380 m²/g, 12 % Wasser) in 0,3 l deionisiertem Wasser werden bei RT unter Rühren 100 mg H(AuCl₄) x H₂O, gelöst in 100 ml deionisiertem Wasser, innerhalb von 60 min zugetropft. Zur Fällung des Goldhydroxides wird mit einer 0,5 molaren Na₂CO₃-Lösung der pH-Wert auf 8 eingestellt; die schwach gelbe Suspension entfärbt sich. Die Suspension wird 3 h bei RT gerührt, der Feststoff abgetrennt und 4 mal mit je 25 ml VE-Wasser gewaschen. Zur Trocknung wird der Feststoff 2 h bei 150°C und 1 h bei 200°C gehalten, und anschließend wird der getrocknete Kontakt an der Luft 2 h bei 250°C und 5 h bei 400°C kalziniert.

Man erhält einen Katalysator mit 0,5 Gew.-% Gold. Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 2

Katalysatorpräparation analog Beispiel 1, aber die wäßrige Suspension wird vor der Goldzugabe auf 80 °C aufgeheizt.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 3

Katalysatorpräparation analog Beispiel 1, aber es werden 200 mg H(AuCl₄), gelöst in 100 ml VE-Wasser, zugesetzt. Man erhält einen Katalysator mit 1,0 Gew.-% Gold. Die Charakterisierung mit TEM ergibt nanoskalige Goldpartikel mit mittleren Teilchendurchmessern von ca. 3-6 nm.

Ergebnisse der katalytischen Reaktion analog den Standardreaktionsbedingungen sind in Tabelle 1 zusammengefaßt.

### Beispiel 4

Katalysatorpräparation analog Beispiel 1, aber es werden 0,5 h nach der Goldhydroxid-Fällung 464 mg Mononatriumcitrat zugesetzt.

Man erhält einen Katalysator mit 0,5 Gew.-% Gold. Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 5

Katalysatorpräparation analog Beispiel 1, aber es werden 0,5 h nach der Goldhydroxid-Fällung 464 mg Magnesiumcitrat zugesetzt.

Man erhält einen Katalysator mit 0,5 Gew.-% Gold. Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 6

Katalysatorpräparation analog Beispiel 1, aber der getrocknete Kontakt wird nur 1 h bei 250°C kalziniert. 10 g dieses getrockneten Katalysators werden erneut analog Beispiel 1 mit 100 mg H(AuCl₄) umgesetzt, gewaschen, getrocknet und kalziniert.

Man erhält einen Katalysator mit 1,0 Gew.-% Gold. Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 7

Katalysatorpräparation analog Beispiel 1, aber das Titanoxidhydrat enthält 0,1 % Sulfat und 12 % Wasser.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 8

Katalysatorpräparation analog Beispiel 1, aber das Titanoxidhydrat enthält 4 % Sulfat und 12 % Wasser.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 9

Katalysatorpräparation analog Beispiel 1, aber die Kalzinierung wird 5 h bei 500 °C durchgeführt.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 10

Katalysatorpräparation analog Beispiel 1, aber der pH Wert wird auf 7,0 eingestellt.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 11

Katalysatorpräparation analog Beispiel 1, aber aber der pH Wert wird auf 7,5 eingestellt.

Man erhält einen Katalysator mit 0,5 Gew.-% Gold. Die Charakterisierung mit TEM ergibt nanoskalige Goldpartikel mit mittleren Teilchendurchmessern von ca. 3-8 nm.

Ergebnisse der katalytischen Reaktion analog den Standardreaktionsbedingungen sind in Tabelle 1 zusammengefaßt.

### Beispiel 12

Katalysatorpräparation analog Beispiel 1, aber es wird ZnO anstelle von Titanoxidhydrat eingesetzt.

Man erhält einen Gold-Zinkoxid-Katalysator mit 0,5 Gew.-% Gold. Die Charakterisierung mit TEM ergibt nanoskalige Goldpartikel mit mittleren Teilchen-durchmessern von ca. 3-10 nm.

Ergebnisse der katalytischen Reaktion analog den Standardreaktionsbedingungen sind in Tabelle 1 zusammengefaßt.

### Beispiel 13

Katalysatorpräparation analog Beispiel 1, aber es wird ZnO / 3% TiO₂ (Anatas) anstelle von Titanoxidhydrat eingesetzt.

Man erhält einen Gold-Zinkoxid/Titandioxid-Katalysator mit 0,5 Gew.-% Gold. Die Charakterisierung mit TEM ergibt nanoskalige Goldpartikel mit mittleren Teilchendurchmessern von ca. 3-10 nm.

Ergebnisse der katalytischen Reaktion analog den Standardreaktionsbedingungen sind in Tabelle 1 zusammengefaßt.

### Beispiel 14

100 mg H(AuCl₄) werden in 50 ml VE-Wasser gelöst und 10 g Titanoxidhydrat unter Rühren eingetragen und die Suspension mit 0,5 molarer Na₂CO₃-Lösung auf pH 8 eingestellt. Anschließend wird 464 mg Magnesiumcitrat zugesetzt. Nach 2 h Rühren wird der Feststoff abgetrennt und 5 mal mit 100 ml Wasser gewaschen, 2 h bei 150°C getrocknet und 5 h bei 400°C kalziniert.

Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 15

Katalysatorpräparation analog Beispiel 14, aber die Oxidationsreaktion erfolgt bei 40°C.

### Beispiel 16

Katalysatorpräparation analog Beispiel 14, aber die Oxidationsreaktion erfolgt bei 30°C.

### Beispiel 17

Katalysatorpräparation analog Beispiel 16, aber der getrocknete Kontakt wird nur 1 h bei 250°C kalziniert. 10 g dieses getrockneten Katalysators werden erneut analog Beispiel 16 mit 100 mg H(AuCl₄) umgesetzt, gewaschen, getrocknet und kalziniert.

Man erhält einen Katalysator mit 1,0 Gew.-% Gold. Die Charakterisierung mit TEM ergibt nanoskalige Goldpartikel mit mittleren Teilchendurchmessern von ca. 1-6 nm.

Ergebnisse der katalytischen Reaktion analog den Standardreaktionsbedingungen sind in Tabelle 1 zusammengefaßt.

### Beispiel 18

Katalysatorpräparation analog Beispiel 1, aber die Reaktion analog den Standardreaktionsbedingungen wird bei 3 bar Überdruck durchgeführt.

Ergebnisse der katalytischen Reaktion analog den Standardreaktionsbedingungen sind in Tabelle 1 zusammengefaßt.

### Beispiel 19

Katalysatorpräparation analog Beispiel 1, aber die Reaktion analog den Standardreaktionsbedingungen wird bei 10 bar Überdruck durchgeführt.

Ergebnisse der katalytischen Reaktion analog den Standardreaktionsbedingungen sind in Tabelle 1 zusammengefaßt.

### Beispiel 20

Katalysatorpräparation analog Beispiel 14, aber es wird bei den Standardreaktionsbedingungen Ethylen anstelle von Propen eingesetzt.

Ergebnisse der katalytischen Reaktion analog den Standardreaktionsbedingungen sind in Tabelle 1 zusammengefaßt.

### Beispiel 21

Katalysatorpräparation analog Beispiel 14, aber es wird bei den Standardreaktionsbedingungen 1-Buten eingesetzt.

### Vergleichsversuch 1:

Eine Lösung von 0,104 g HAuCl₄ x 4 H₂O in 400 ml destilliertem Wasser wird auf 70°C aufgeheizt, mit einer wäßrigen 0,1 N NaOH-Lösung auf pH 7,5 gebracht und bei intensiven Rühren 5 g Titandioxid (Anatas-Rutil-Mischoxid; P 25 der Firma Degussa) in einer Portion hinzugefügt und 1 h weiter gerührt. Der Feststoff wird 5 mal mit je 3 Liter destilliertem Wasser gewaschen, bei Raumtemperatur im Vakuum für 12 Stunden getrocknet und 4 h bei 400 °C kalziniert. Man erhält einen Gold-Titandioxid-Katalysator mit 1 Gew.-% Gold.

Ergebnisse der katalytischen Reaktion analog den Standardreaktionsbedingungen sind in Tabelle 1 zusammengefaßt.

### Vergleichsversuch 2:

Zu 200 ml einer wäßrigen Lösung, welche hierin aufgelöst 22,3 g (0,21 mol) Natriumcarbonat enthielt, wurden tropfenweise 300 ml einer wäßrigen Lösung, welche 50 g (0,05 mol) einer wäßrigen 24 Gew.-%igen Lösung von Ti(SO₄)₂ (berechnet auf Titanylsulfat: Ti(SO₄)₂ + H₂O = TiOSO₄ + H₂SO₄) und 1,08 g HAuCl₄ hierin aufgelöst enthielt, während einer Zeitspanne von 30 min zugesetzt. 5 Minuten nach Abschluß der tropfenweisen Zugabe wurden die erhaltene wäßrige Suspension des Mischniederschlages und 400 ml einer gesättigten wäßrigen Lösung von Magnesiumcitrat (6 g/l) fortlaufend während 1 h zur Alterung gerührt. Der pH-Wert ist 8,3. Nach Waschung des Niederschlags wird der Feststoff im Vakuum getrocknet und 5 h bei 400 °C kalziniert.
Man erhält einen Gold-Titandioxid-Katalysator mit 11 Gew.-% Gold.

Ergebnisse der katalytischen Reaktion analog den Standardreaktionsbedingungen sind in Tabelle 1 zusammengefaßt.

**Tabelle 1**

| Katalysator | Propenoxid Selektivität (%) | Propenoxid Ausbeute (%) |
|---|---|---|
| Beispiel 1 | > 97 | 5,1 |
| Beispiel 2 | > 97 | 4,7 |
| Beispiel 3 | > 97 | 3,4 |
| Beispiel 4 | > 97 | 5,6 |
| Beispiel 5 | > 97 | 5,6 |
| Beispiel 6 | > 97 | 5,8 |
| Beispiel 7 | > 97 | 4,0 |
| Beispiel 8 | > 97 | 3,7 |
| Beispiel 9 | > 97 | 5,4 |
| Beispiel 10 | > 97 | 3,5 |
| Beispiel 11 | > 97 | 4,4 |
| Beispiel 12 | > 97 | 0,4 |
| Beispiel 13 | > 97 | 0,9 |
| Beispiel 14 | > 97 | 5,7 |
| Beispiel 15 | > 97 | 5,1 |
| Beispiel 16 | > 97 | 4,6 |
| Beispiel 17 | > 97 | 5,7 |
| Beispiel 18 | > 97 | 5,7 |
| Beispiel 19 | > 97 | 4,9 |
| Beispiel 20 | > 97 | 5,0 |
| Beispiel 21 | > 97 | 5,5 |
| Vergleichsversuch 1 | > 97 | 1,4 |
| Vergleichsversuch 2 | > 97 | 0,5 |

## Patentansprüche

1. Verfahren zur Oxidation ungesättigter Kohlenwasserstoffe in der Gasphase in Gegenwart eines Wasserstoff-Sauerstoff-Gemisches an einem mit Goldteilchen belegten Katalysator, **dadurch gekennzeichnet, daß** ein mit nanoskaligen Goldteilchen belegter aus gegebenenfalls dotiertem Titanoxidhydrat hergestellter und kalzinierter Katalysator eingesetzt wird.

2. Verfahren nach Anspruch 1, **dadurch gekennzeichnet, daß** der Katalysator nach der "deposition-precipitation"-Methode hergestellt wird.

3. Verfahren nach einem der Ansprüche 1 oder 2, **dadurch gekennzeichnet, daß** ein aus mit Sulfat in einer Menge zwischen 0,05 und 10 Gew.-% dotiertem Titanoxidhydrat hergestellter und kalzinierter Katalysator eingesetzt wird.

4. Verfahren nach einem der Ansprüche 1 bis 3, **dadurch gekennzeichnet, daß** ein mit nanoskaligen Goldteilchen im Bereich zwischen 1 bis 10 nm belegter Katalysator eingesetzt wird.

5. Verfahren nach einem der Ansprüche 1 bis 4, **dadurch gekennzeichnet, daß** die Oxidation ungesättigter Kohlenwasserstoffe gegebenenfalls in Gegenwart eines Inertgases bei einem molaren Verhältnis des eingesetzten Gasgemisches Wasserstoff/ ungesättigter Kohlenwasserstoff/ Sauerstoff / Inertgas von 20 bis 80 % / 10 bis 50 % / 1 bis 10 % / 0 bis 50 % durchgeführt wird.

6. Verfahren nach einem der Ansprüche 1 bis 5, **dadurch gekennzeichnet, daß** als ungesättigter Kohlenwasserstoff Ethen, Propen, 1-Buten oder 2-Buten eingesetzt wird.

7. Mit Goldteilchen belegter Trägerkatalysator zur Oxidation ungesättigter Kohlenwasserstoffe, dadurch erhalten, daß ein gegebenenfalls Sulfat enthaltendes Titanoxidhydrat nach der "deposition-precipitation"-Methode mit nanoskalierten Goldteilchen in einer Menge von 0,05 bis 10 Gew.-%, bezogen auf die Trägersubstanz, belegt und anschließend gewaschen, getrocknet und kalziniert wird.

8. Katalysator nach Anspruch 7, **dadurch gekennzeichnet, daß** der Katalysator vor der Kalzinierung mehrfach mit nanoskalierten Goldteilchen nach der "deposition-precipitation"-Methode belegt wird.

## Claims

1. A process for the oxidation of an unsaturated hydrocarbon in the gas phase in the presence of a hydrogen/oxygen mixture on a catalyst coated with gold particles, **characterized in that** a calcined catalyst which has been prepared from optionally doped titanium oxide hydrate and is coated with nanoscale gold particles is employed.

2. A process according to claim 1, **characterized in that** the catalyst is prepared by the "deposition-precipitation" method.

3. A process according to either of claims 1 or 2, **characterized in that** a calcined catalyst which has been prepared from titanium oxide hydrate doped with. sulfate in an amount of between 0.05 and 10 wt.% is employed.

4. A process according to any one of claims 1 to 3, **characterized in that** a catalyst coated with nanoscale gold particles in the range between 1 to 10 nm is employed.

5. A process according to any one of claims 1 to 4, **characterized in that** the oxidation of unsaturated hydrocarbons is optionally carried out in the presence of an inert gas at a molar ratio of the gas mixture employed of hydrogen/unsaturated hydrocarbon/oxygen/inert gas of 20 to 80%/10 to 50%/ 1 to 10%/0 to 50%.

6. A process according to any one of claims 1 to 5, **characterized in that** ethene, propene, 1-butene or 2-butene is employed as the unsaturated hydrocarbon.

7. A supported catalyst coated with gold particles for the oxidation of unsaturated hydrocarbons, obtainable by a procedure in which a titanium oxide hydrate, which optionally contains sulfate, is coated with nanoscale gold particles in an amount of 0.05 to 10 wt.%, based on the support substance, by the "deposition-precipitation" method, and then washed, dried and calcined.

8. A catalyst according to claim 7, **characterized in that** before the calcining, the catalyst is coated with nanoscale gold particles several times by the "deposition-precipitation" method.

## Revendications

1. Procédé pour l'oxydation d'hydrocarbures insaturés en phase gazeuse en présence d'un mélange d'hydrogène - oxygène sur un catalyseur enduit de particules d'or, **caractérisé en ce qu'**on met en oeuvre un catalyseur enduit de particules d'or de la dimension du nanomètre, préparé à partir d'oxydhydrate de titane le cas échéant dopé, et calciné.

2. Procédé selon la revendication 1, **caractérisé en ce qu'**on prépare le catalyseur conformément au procédé de "déposition - précipitation".

3. Procédé selon l'une quelconque des revendications 1 ou 2, **caractérisé en ce qu'**on met en oeuvre un catalyseur préparé à partir d'oxydhydrate de titane dopé avec du sulfate en une quantité entre 0,05 et 10 % en poids et calciné.

4. Procédé selon l'une quelconque des revendications 1 à 3, **caractérisé en ce qu'**on met en oeuvre un catalyseur enduit de particules d'or de la dimension du nanomètre dans le domaine de 1 à 10 nm.

5. Procédé selon l'une quelconque des revendications 1 à 4, **caractérisé en ce qu'**on effectue l'oxydation d'hydrocarbures insaturés, le cas échéant en présence d'un gaz inerte, à un rapport molaire du mélange de gaz mis en oeuvre hydrogène / hydrocarbure insaturé / oxygène / gaz inerte de 20 à 80 % / de 10 à 50 % / de 1 à 10 % / de 0 à 50 %.

6. Procédé selon l'une quelconque des revendications 1 à 5, **caractérisé en ce qu'**on met en oeuvre, à titre d'hydrocarbure insaturé, de l'éthène, du propène, du 1-butène ou du 2-butène.

7. Catalyseur déposé sur un support, enduit de particules d'or, pour l'oxydation d'hydrocarbures insaturés, que l'on obtient en recouvrant un oxydhydrate de titane contenant le cas échéant du sulfate, conformément au procédé de "déposition-précipitation" avec des particules d'or de la dimension du nanomètre, en une quantité de 0,05 à 10 % en poids, rapportée à la substance de support, puis on le lave, on le sèche et on le calcine.

8. Catalyseur selon la revendication 7, **caractérisé en ce qu'**on recouvre le catalyseur avant la calcination à plusieurs reprises avec des particules d'or de la dimension du nanomètre conformément au procédé de "déposition-précipitation".
